# EUROPÄISCHE PATENTSCHRIFT

(11) **EP 0 828 728 B1**
(45) Veröffentlichungstag und Bekanntmachung des Hinweises auf die Patenterteilung: **22.01.2003**
(21) Anmeldenummer: 96916048.0
(22) Anmeldetag: 11.05.1996
(51) Int. Cl.: C07D 307/94, C07D 307/86, A61K 31/34

(54) **PHENYLDIHYDROBENZOFURANE**
PHENYL DIHYDROBENZOFURANES
DIHYDROBENZOFURANES DE PHENYLE

(30) Priorität: 18.05.1995 CH 147295
(43) Veröffentlichungstag der Anmeldung: 18.03.1998
(73) Patentinhaber: ALTANA Pharma AG, 78467 Konstanz (DE)
(72) Erfinder: AMSCHLER, Hermann, "" (DE); BÄR, Thomas, D-78479 Reichenau (DE); GUTTERER, Beate, D-78467 Allensbach (DE); FLOCKERZI, Dieter, D-78476 Allensbach (DE); MARTIN, Thomas, D-78462 Konstanz (DE); THIBAUT, Ulrich, D-78464 Konstanz (DE); HATZELMANN, Armin, D-78467 Konstanz (DE); BEUME, Rolf, D-78465 Konstanz (DE); BOSS, Hildegard, D-78476 Allensbach (DE); HÄFNER, Dietrich, D-78464 Konstanz (DE); KLEY, Hans-Peter, D-78476 Allensbach (DE); ULRICH, Wolf-Rüdiger, D-78464 Konstanz (DE)
(86) Internationale Anmeldenummer: EP9602031
(87) Internationale Veröffentlichungsnummer: WO96036625

(56) Entgegenhaltungen:
- WO-A-93/19747
- WO-A-93/19749
- WO-A-93/19751
- WO-A-94/02465

## Beschreibung

### Technisches Gebiet

Die Erfindung betrifft neue Verbindungen, die in der pharmazeutischen Industrie zur Herstellung von Medikamenten verwendet werden.

### Stand der Technik

In der internationalen Patentanmeldung W092/12961 werden Benzamide mit PDE-hemmenden Eigenschaften beschrieben. In der internationalen Patentanmeldung W093/25517 werden trisubstituierte Phenylderivate als selektive PDE-IV-Hemmer offenbart. In der internationalen Patentanmeldung W094/02465 werden Inhibitoren der c-AMP Phosphodiesterase und des TNF beschrieben.

### Beschreibung der Erfindung

Es wurde nun gefunden, daß die nachfolgend näher beschriebenen Phenyldihydrobenzofurane, die sich von den vorveröffentlichten Verbindungen durch eine völlig andersartige Substitution unterscheiden, überraschende und besonders vorteilhafte Eigenschaften besitzen.

Gegenstand der Erfindung sind somit Verbindungen der Formel I (siehe beigefügtes Formelblatt I), worin
- R1: 1-6C-Alkoxy, 3-7C-Cycloalkoxy, 3-7C-Cycloalkylmethoxy, Benzyloxy oder ganz oder teilweise durch Fluor substituiertes 1-4C-Alkoxy bedeutet,
- R2: 1-4C-Alkyl und
- R3: Wasserstoff oder 1-4C-Alkyl bedeutet,
oder
- R2 und R3: gemeinsam und unter Einschluß der beiden Kohlenstoffatome, an die sie gebunden sind, einen 5-, 6- oder 7-gliedrigen, gewünschtenfalls durch ein Sauerstoffatom unterbrochenen Kohlenwasserstoffring darstellen,
- R4: Wasserstoff, Hydroxy, Carboxyl, 1-4C-Alkoxycarbonyl, Amino, Mono- oder Di-1-4C-alkylamino, 1-4C-Alkylcarbonylamino, 1-4C-Alkylsulfonylamino, Trifluormethylsulfonylamino, Cyanamino, Nitro, Cyano, Mono- oder Di-1-4C-alkylaminocarbonyl, 5-Tetrazolyl oder Carbamoyl und
- R5: Wasserstoff, Hydroxy, 1-4C-Alkoxy oder Halogen bedeutet und
wobei mindestens einer der Reste R3, R4 und R5 eine von Wasserstoff verschiedene Bedeutung hat,
und deren Salze.

1-6C-Alkoxy steht für einen Rest, der neben dem Sauerstoffatom einen geradkettigen oder verzweigten Alkylrest mit 1 bis 6 Kohlenstoffatomen enthält. Als Alkylreste mit 1 bis 6 Kohlenstoffatomen seien hierbei beispielsweise genannt der Hexyl-, Isohexyl- (2-Methylpentyl-), Neohexyl- (2,2-Dimethylbutyl-), Pentyl-, Isopentyl- (3-Methylbutyl-), Neopentyl- (2,2-Dimethylpropyl-), Butyl-, iso-Butyl-, sec.-Butyl-, tert.-Butyl-, Propyl-, Isopropyl-, Ethyl- und der Methylrest.

3-7C-Cycloalkoxy steht beispielsweise für Cyclopropyloxy, Cyclobutyloxy, Cyclopentyloxy, Cyclohexyloxy und Cycloheptyloxy, wovon Cyclopropyloxy, Cyclobutyloxy und Cyclopentyloxy bevorzugt sind.

3-7C-Cycloalkylmethoxy steht beispielsweise für Cyclopropylmethoxy, Cyclobutylmethoxy, Cyclopentylmethoxy, Cyclohexylmethoxy und Cycloheptylmethoxy, wovon Cyclopropylmethoxy, Cyclobutylmethoxy und Cyclopentylmethoxy bevorzugt sind.

Als ganz oder teilweise durch Fluor substituiertes 1-4C-Alkoxy seien beispielsweise der 1,2,2-Trifluorethoxy-, der 2,2,3,3,3-Pentafluorpropoxy-, der Perfluorethoxy- und insbesondere der 1,1,2,2-Tetrafluorethoxy-, der Trifluormethoxy-, der 2,2,2-Trifluorethoxy- und der Difluormethoxyrest genannt.

Als 5-, 6- oder 7-gliedriger, gewünschtenfalls durch ein Sauerstoffatom unterbrochener Kohlenwasserstoffring seien der Cyclopentan-, der Cyclohexan-, der Cycloheptan-, der Tetrahydrofuran- und der Tetrahydropyranring genannt. Wenn R2 und R3 gemeinsam und unter Einschluß der beiden Kohlenstoffatome, an die sie gebunden sind, einen 5-, 6- oder 7-gliedrigen Ring bilden, so liegt eine Spiroverbindung vor.

1-4C-Alkyl steht für geradkettige oder verzweigte Alkylreste mit 1 bis 4 Kohlenstoffatomen. Beispielsweise seien genannt der Butyl-, iso-Butyl-, sec.-Butyl-, tert.-Butyl-, Propyl-, Isopropyl-, Ethyl- und der Methylrest.

1-4C-Alkoxy steht für einen Rest, der neben dem Sauerstoffatom einen der vorstehend genannten 1-4C-Alkylreste enthält. Beispielsweise seien der Methoxy- und der Ethoxyrest genannt.

1-4C-Alkoxycarbonyl steht für eine Carbonylgruppe, an die einer der vorstehend genannten 1-4C-Alkoxyreste gebunden ist. Beispielsweise seien der Methoxycarbonyl- (CH₃O-CO-) und der Ethoxycarbonylrest (CH₃CH₂O-CO-) genannt.

Als Mono- oder Di-1-4C-alkylaminoreste seien beispielsweise der Methylamino-, der Dimethylamino-, der Ethylamino-, der Diethylamino-, der Propylamino- und der Isopropylaminorest genannt.

Als 1-4C-Alkylcarbonylaminorest sei beispielsweise der Acetylaminorest (-NH-CO-CH₃) genannt.

1-4C-Alkylsulfonylamino steht für einen Sulfonylaminorest der durch einen der vorstehend genannten 1-4C-Alkylreste substituiert ist. Beispielsweise sei der Methylsulfonylaminorest (-NH-SO₂-CH₃) genannt.

Unter Halogen werden erfindungsgemäß Fluor, Chlor, Brom und Iod, vorzugsweise Fluor und Chlor, verstanden.

Mono- und Di-1-4C-alkylaminocarbonyl enthalten neben der Carbonylgruppe einen der vorstehend genannten Mono- bzw. Di-1-4C-alkylaminoreste. Beispielsweise genannt seien der N-Methyl-, der N,N-Dimethyl-, der N-Ethyl-, der N-Propyl-, der N,N-Diethyl- und der N-Isopropylcarbamoylrest.

Hervorzuhebende Verbindungen der Formel I sind solche, in denen
- R1: 1-4C-Alkoxy, 3-5C-Cycloalkoxy, 3-5C-Cycloalkylmethoxy oder ganz oder teilweise durch Fluor substituiertes 1-4C-Alkoxy bedeutet,
- R2: 1-4C-Alkyl und
- R3: Wasserstoff oder 1-4C-Alkyl bedeutet,
oder
- R2 und R3: gemeinsam und unter Einschluß der beiden Kohlenstoffatome, an die sie gebunden sind, einen Cyclopentan-, Cyclohexan-, Tetrahydrofuran- oder Tetrahydropyranring darstellen,
- R4: Wasserstoff, Hydroxy, Carboxyl, 1-2C-Alkoxycarbonyl, Nitro, Cyano, Mono- oder Di-1-4C-alkylaminocarbonyl, 5-Tetrazolyl oder Carbamoyl und
- R5: Wasserstoff, Hydroxy, Methoxy oder Ethoxy bedeutet und
wobei mindestens einer der Reste R3, R4 und R5 eine von Wasserstoff verschiedene Bedeutung hat,
und deren Salze.

Bevorzugte Verbindungen der Formel I sind solche, in denen
- R1: 1-4C-Alkoxy, 3-5C-Cycloalkoxy oder ganz oder teilweise durch Fluor substituiertes 1-2C-Alkoxy bedeutet,
- R2 und R3: gemeinsam und unter Einschluß der beiden Kohlenstoffatome, an die sie gebunden sind, einen Cyclopentan- oder Cyclohexanring darstellen,
- R4: Wasserstoff, Hydroxy, Carboxyl, 1-2C-Alkoxycarbonyl, Cyano, Mono- oder Di-1-4C-alkylaminocarbonyl, 5-Tetrazolyl oder Carbamoyl und
- R5: Wasserstoff bedeutet,
und deren Salze.

Besonders bevorzugte Verbindungen der Formel I sind solche, in denen
- R1: Methoxy,
- R2 und R3: gemeinsam und unter Einschluß der beiden Kohlenstoffatome, an die sie gebunden sind, einen Cyclopentanring darstellen,
- R4: Hydroxy, Carboxyl, Cyano, Mono-1-4C-alkylaminocarbonyl, 5-Tetrazolyl oder Carbamoyl und
- R5: Wasserstoff bedeutet,
und deren Salze.

Als Salze kommen für Verbindungen der Formel I - je nach Substitution- alle Säureadditionssalze oder alle Salze mit Basen in Betracht. Besonders erwähnt seien die pharmakologisch verträglichen Salze der in der Galenik üblicherweise verwendeten anorganischen und organischen Säuren. Als solche eignen sich einerseits wasserlösliche und wasserunlösliche Säureadditionssalze mit Säuren wie beispielsweise Salzsäure, Bromwasserstoffsäure, Phosphorsäure, Salpetersäure, Schwefelsäure, Essigsäure, Zitronensäure, D-Gluconsäure, Benzoesäure, 2-(4-Hydroxybenzoyl)-benzoesäure, Buttersäure, Sulfosalicylsäure, Maleinsäure, Laurinsäure, Äpfelsäure, Fumarsäure, Bernsteinsäure, Oxalsäure, Weinsäure, Embonsäure, Stearinsäure, Toluolsulfonsäure, Methansulfonsäure oder 3-Hydroxy-2-naphthoesäure, wobei die Säuren bei der Salzherstellung - je nachdem, ob es sich um eine ein- oder mehrbasige Säure handelt und je nachdem, welches Salz gewünscht wird - im äquimolaren oder einem davon abweichenden Mengenverhältnis eingesetzt werden.

Andererseits kommen auch Salze mit Basen in Betracht. Als Beispiele für Salze mit Basen seien Alkali- (Lithium-, Natrium-, Kalium-) oder Calcium-, Aluminium-, Magnesium-, Titan-, Ammonium-, Meglumin- oder Guanidiniumsalze erwähnt, wobei auch hier bei der Salzherstellung die Basen im äquimolaren oder einem davon abweichenden Mengenverhältnis eingesetzt werden.

Pharmakologisch unverträgliche Salze, die beispielsweise bei der Herstellung der erfindungsgemäßen Verbindungen im industriellen Maßstab als Verfahrensprodukte zunächst anfallen können, werden durch dem Fachmann bekannte Verfahren in pharmakologisch verträgliche Salze übergeführt.

Bei den Verbindungen der Formel I handelt es sich - sofern die Substitutionen -R2 und -CH₂R3 nicht identisch sind - um chirale Verbindungen. Die Erfindung umfaßt daher sowohl die reinen Enantiomeren als auch deren Gemische in jedem Mischungsverhältnis, einschließlich der Racemate. Die Enantiomeren können in an sich bekannter Weise (beispielsweise durch Herstellung und Trennung entsprechender diastereoisomerer Verbindungen) separiert werden.

Weiterer Gegenstand der Erfindung ist ein Verfahren zur Herstellung der Verbindungen der Formel I und ihrer Salze. Das Verfahren ist dadurch gekennzeichnet, daß man
a) Verbindungen der Formel II (siehe beigefügtes Formelblatt I), in denen R1, R2, R3 und R5 die oben angegebenen Bedeutungen haben, katalytisch dehydriert oder daß man
b) Verbindungen der Formel III (siehe beigefügtes Formelblatt I), in denen R1, R2 und R3 die oben genannten Bedeutungen besitzen und X die Gruppe -B(OH)₂ bedeutet mit einer Verbindung der Formel IV (siehe beigefügtes Formelblatt I), worin R4 und R5 die oben genannten Bedeutungen besitzen und Z Halogen bedeutet, katalytisch kuppelt oder daß man
c) Verbindungen der Formel I, in denen R1, R2, R3 und R5 die oben angegebenen Bedeutungen haben und R4 Carboxyl bedeutet, mit Aminen der Formel H-N(R11)R12, worin R11 und R12 unabhängig voneinander Wasserstoff oder 1-4C-Alkyl bedeuten, umsetzt, oder daß man
d) zur Herstellung von Verbindungen der Formel I, in denen R1, R2, R3 und R5 die oben angegebenen Bedeutungen haben und R4 Cyano bedeutet, entsprechende Verbindungen der Formel I worin R4 Carbamoyl bedeutet dehydratisiert, oder daß man
e) zur Herstellung von Verbindungen der Formel I, in denen R1, R2, R3 und R5 die oben angegebenen Bedeutungen haben und R4 5-Tetrazolyl bedeutet, entsprechende Verbindungen der Formel I worin R4 Cyano bedeutet mit einem Azidderivat cycloaddiert,
und daß man gewünschtenfalls anschließend nach a), b), c), d) oder e) erhaltene Verbindungen der Formel I in ihre Salze überführt, oder daß man erhaltene Salze der Verbindungen der Formel I in die freien Verbindungen überführt.

Gewünschtenfalls können weitere Verbindungen der Formel I in einer dem Fachmann bekannten Weise durch Derivatisierung (insbesondere der Reste R4) in andere Verbindungen der Formel I übergeführt werden (beispielsweise durch Synthese entsprechender Ester aus den Säuren).

Die Isolierung und Reinigung der erfindungsgemäßen Substanzen erfolgt in an sich bekannter Weise z.B. derart, daß man das Lösungsmittel im Vakuum abdestilliert und den erhaltenen Rückstand aus einem geeigneten Lösungsmittel umkristallisiert oder einer der üblichen Reinigungsmethoden, wie beispielsweise der Säulenchromatographie an geeignetem Trägermaterial, unterwirft.

Salze erhält man durch Auflösen der freien Verbindung in einem geeigneten Lösungsmittel, z.B. in einem chlorierten Kohlenwasserstoff, wie Methylenchlorid oder Chloroform, oder einem niedermolekularen aliphatischen Alkohol (Ethanol, Isopropanol), das die gewünschte Säure bzw. Base enthält, oder dem die gewünschte Säure bzw. Base anschließend zugegeben wird. Die Salze werden durch Filtrieren, Umfällen, Ausfällen mit einem Nichtlösungsmittel für das Anlagerungssalz oder durch Verdampfen des Lösungsmittels gewonnen. Erhaltene Salze können durch Alkalisierung bzw. durch Ansäuern in die freien Verbindungen umgewandelt werden, welche wiederum in Salze übergeführt werden können. Auf diese Weise lassen sich pharmakologisch nicht verträgliche Salze in pharmakologisch verträgliche Salze umwandeln.

Die katalytische Dehydrierung von Verbindungen der Formel II nach Variante a) erfolgt in einer dem Fachmann bekannten Weise in einem geeigneten Lösungsmittel (beispielsweise Toluol) und in Gegenwart eines geeigneten Katalysators wie Palladium auf Aktivkohle, vorzugsweise bei erhöhter Temperatur, insbesondere bei der Siedetemperatur des Lösungsmittels.

Die katalytische Kupplung der Boronsäure der Formel III mit dem Arylhalogenid der Formel IV [Variante b)] erfolgt unter Bedingungen, wie sie dem Fachmann bekannt sind, beispielsweise so wie in den nachfolgenden Beispielen beschrieben. Als Arylhalogenide werden dabei vorzugsweise Verbindungen der Formel IV eingesetzt, worin Z die Bedeutung Brom hat.

Die Umsetzung von Verbindungen der Formel I nach Variante c) erfolgt in einer Weise, wie sie dem Fachmann zur Synthese von Carbonsäureamiden bekannt ist. Gewünschtenfalls wird die Carbonsäure der Formel I vor der Aminolyse in ein geeignet aktiviertes Derivat, beispielsweise ein entsprechendes Säurehalogenid, übergeführt. Als Amine der Formel H-N(R11)R12 die eingesetzt werden seien beispielsweise Ammoniak, Methylamin und Ethylamin genannt.

Die Dehydratisierung analog Variante d) erfolgt ebenfalls auf bekannte Weise in einem geeigneten Lösungsmittel durch Behandlung mit wasserentziehenden Mitteln wie Phosphoroxychlorid oder Phosphorpentoxid, vorzugsweise bei erhöhter Temperatur, insbesondere bei der Siedetemperatur des verwendeten Lösungsmittels.

Die Bedingungen der Cycloaddition nach Variante e) sind dem Fachmann ebenfalls bekannt. Als geeignetes Azidderivat wird beispielsweise Tributylzinnazid verwendet.

Verbindungen der Formel II, worin R1, R2, R3 und R5 die oben genannten Bedeutungen haben, sind beispielsweise durch Addition entsprechender Verbindungen der Formel III, worin X die Bedeutung Lithium hat, an Verbindungen der Formel V (siehe beigefügtes Formelblatt I), worin R5 die oben genannten Bedeutungen hat, und anschließende Eliminierung von Wasser zugänglich. Zweckmäßigerweise werden Verbindungen der Formel V in teilweise geschützter Form, beispielsweise als Monoethylenketal, eingesetzt und die Schutzgruppe nach erfolgter Umsetzung wieder abgespalten.

Verbindungen der Formel III worin X Lithium bedeutet, sind aus entsprechenden Verbindungen der Formel III worin X Halogen, insbesondere Brom, bedeutet durch Metall-Halogen-Austausch zugänglich.

Verbindungen der Formel III, worin X die Gruppe -B(OH)₂ bedeutet sind aus entsprechenden Verbindungen der Formel III, worin X Lithium bedeutet, durch Umsetzung mit einem Borsäuretrialkylester und anschließender Hydrolyse zugänglich.

Die Verbindungen der Formel III worin X Halogen bedeutet, können gemäß dem allgemeinen Reaktionsschema auf dem beigefügten Formelblatt II hergestellt werden. Die Synthese von Verbindungen der Formel III ist beispielhaft unter "Ausgangsverbindungen" beschrieben. Weitere Verbindungen der Formel III können in analoger Weise hergestellt werden.

Verbindungen der Formeln IV und V sind bekannt oder können auf bekannte Weise hergestellt werden.

Die folgenden Beispiele erläutern die Erfindung näher, ohne sie einzuschränken. In den Beispielen steht Schmp. für Schmelzpunkt, Sdp. für Siedepunkt, h für Stunde(n), RT für Raumtemperatur, THF für Tetrahydrofuran, DMF für N,N-Dimethylformamid und DC für Dünnschichtchromatographie. Die in den Beispielen genannten Verbindungen und ihre Salze sind bevorzugter Gegenstand der Erfindung.

### Beispiele

### Endprodukte

### 1. 4-(2,3-Dihydro-7-methoxybenzofuran-2-spiro-1'-cyclopentan-4-yl)phenol

Zu einer Lösung von 0,3 g (1 mmol) der nach Al hergestellten Verbindung in 5 ml absolutem Toluol gibt man 3 mg 10 %iges Pd/C und kocht für 72 h am Rückfluß. Nach Abkühlen der Lösung wird vom Katalysator abfiltriert und mit ca. 100 ml Methanol nachgewaschen. Die organischen Phasen werden vereinigt, zur Trockne eingeengt, der Rückstand in Ether aufgenommen, die Etherphase mit 100 ml 1N Natronlauge ausgeschüttelt, die organische Phase abgetrennt, die wäßrige Phase mit 6N Salzsäure auf pH 4 gebracht und mit Ethylether ausgeschüttelt. Anschließend wird die Ethylether-Phase über Natriumsulfat getrocknet und am Rotationsverdampfer eingedampft. Säulenchromatographie ergibt 0,15 g der Titelverbindung als weiß-gräulichen Feststoff vom Schmp. 92-93°C.

### 2. 4-(2,3-Dihydro-7-methoxybenzofuran-2-spiro-1'-cyclopentan-4-yl)-benzoesäure

0,41 g 2,3-Dihydro-7-methoxybenzofuran-2-spiro-1'-cyclopentan-4-boronsäure, 0,36 g 4-Brombenzoesäure und 0,59 g Natriumcarbonat werden unter Stickstoff in einem Gemisch aus 12 ml Toluol, 4,8 ml Wasser und 2,4 ml Ethanol bei RT gerührt. Dann werden 0,04 g Tetrakistriphenylphosphinpalladium (0) zugegeben und das Gemisch 22 h zum Sieden erhitzt. Nach Abkühlen werden Ethylacetat und 0,5 N NaOH zugegeben. Die Phasen werden getrennt und die organische Phase noch 2 x mit 0,5 N NaOH ausgeschüttelt. Die vereinigten wäßrigen Phasen werden mit 2 N HCl auf pH 5 angesäuert und der Niederschlag 2 x mit Ethylacetat extrahiert. Die vereinigten organischen Phasen werden mit gesättigter NaCl-Lösun gewaschen, über Magnesiumsulfat getrocknet und eingeengt. Der Rückstand wird in ca. 15 ml Ethylacetat mit Aktivkohle gekocht, heiß über Kieselgel/Celite filtriert und die Lösung abgekühlt. Das auskristallisierte Produkt wird abgesaugt, mit Ether gewaschen und getrocknet. Man erhält 0,14 g der Titelverbindung mit Schmp. 240-242°C.

### 3. 4-(2,3-Dihydro-7-methoxybenzofuran-2-spiro-1'-cyclopentan-4-yl)-benzoesäureamid

0,5 g 4-(2,3-Dihydro-7-methoxybenzofuran-2-spiro-1'-cyclopentan-4-yl)-benzoesäure (Beispiel 2) werden in 5 ml abs. Toluol mit 0,2 ml SOCl₂ versetzt und das Gemisch 2 h zum Sieden erhitzt. Nach Abkühlen wird das Lösungsmittel im Vakuum am Rotationsverdampfer abdestilliert, mit 2 x 5 ml Toluol nachdestilliert und der Rückstand im Vakuum getrocknet. Das rohe Säurechlorid wird in 5 ml abs. Dioxan gelöst und unter Kühlung zu 2 ml konz. Ammoniaklösung getropft. Nach 1,5 h Rühren bei RT wird mit Wasser verdünnt, der Niederschlag abgesaugt, mit Wasser gewaschen und getrocknet. Zur Reinigung wird über eine Kieselgelsäule mit CH₂Cl₂/EtOH (95:5) chromatografiert. Die chromatografisch reinen Fraktionen werden vereinigt, eingeengt und der Rückstand aus Ethanol kristallisiert. Es wird abgesaugt, mit Ethanol gewaschen und getrocknet. Man erhält 0,13 g der Titelverbindung mit Schmp. 243-244°C.

### 4. 4-(2,3-Dihydro-7-methoxybenzofuran-2-spiro-1'-cyclopentan-4-yl)-benzoesäure-N-methylamid

Analog Beispiel 3 wird aus 0,5 g 4-(2,3-Dihydro-7-methoxybenzofuran-2-spiro-1'-cyclopentan-4-yl)-benzoesäure das Säurechlorid hergestellt und zu 5 ml einer gesättigten Lösung von Methylamin in abs. Dioxan unter Kühlung zugetropft. Das Gemisch wird am Rotationsverdampfer eingeengt und der Rückstand zwischen Ethylacetat und Wasser verteilt. Die organische Phase wird noch einmal mit Wasser gewaschen, über Natriumsulfat getrocknet und eingeengt. Der Rückstand wird über eine Kieselgelsäule mit CH₂Cl₂/EtOH (95:5) chromatografiert. Die chromatografisch reinen Fraktionen werden vereinigt, eingeengt, der Rückstand mit Diisopropylether kristallisiert, abgesaugt und getrocknet. Man erhält 0,17 g der Titelverbindung mit Schmp. 143-144°C.

### 5. 4-(2,3-Dihydro-7-methoxybenzofuran-2-spiro-1'-cyclopentan-4-yl)-benzonitril

1,5 g 4-(2,3-Dihydro-7-methoxybenzofuran-2-spiro-1'-cyclopentan-4-yl)-benzoesäureamid (Beispiel 3) wird in 60 ml Acetonitril suspendiert und dann 0,5 ml POCl₃ zugegeben. Das Gemisch wird 1 h unter Rückfluß zum Sieden erhitzt, abgekühlt und auf Eiswasser gegossen. Es wird mit 1N NaOH neutralisiert und mit Ethylacetat extrahiert. Die organische Phase wird mit Wasser gewaschen, über Natriumsulfat getrocknet und eingeengt. Der Rückstand wird über eine Kieselgelsäule mit Petrolether/Ethylacetat (6:1) chromatografiert. Die chromatografisch reinen Fraktionen werden vereinigt, eingeengt und mit Petrolether/Diisopropylether verrieben. Der Feststoff wird abgesaugt und getrocknet. Man erhält 0,74 g der Titelverbindung mit Schmp. 115-116°C (Zers.).

### 6. 5-[4-(2,3-Dihydro-7-methoxybenzofuran-2-spiro-1'-cyclopentan-4-yl)-phenyl]-tetrazol

Eine Lösung von 0,58 g 4-(2,3-Dihydro-7-methoxybenzofuran-2-spiro-1'-cyclopentan-4-yl)-benzonitril (Beispiel 5) und 0,63 g Tributylzinnazid in 20 ml Ethylenglykolmonomethylether wird 4 Tage unter Rückfluß zum Sieden erhitzt. Nach Abkühlen wird mit 35 ml 4 N HCl und 20 ml Toluol versetzt und die Mischung 3,5 h gerührt. Der Feststoff wird abgesaugt, getrocknet, in einem Gemisch aus Petrolether/Ethylacetat (8:2) in der Siedehitze gelöst und heiß filtriert. Der beim Abkühlen auskristallisierte Feststoff wird abgesaugt, gewaschen und getrocknet. Man erhält 0,15 g der Titelverbindung mit Schmp. 188-189°C (Zers.).

### Ausgangsverbindungen

### A1. 4-(2,3-Dihydro-7-methoxybenzofuran-2-spiro-1'-cyclopentan-4-yl)cyclohex-4-en-1-on

Zu einer Lösung von 7,4 g (0,021 mol) der nach A2 hergestellten Verbindung in 100 ml Toluol gibt man 50 ml destilliertes Wasser und 2 Spatelspitzen p-Toluolsulfonsäuremonohydrat und kocht für 2 h am Rückfluß. Nach Abkühlen wird die organische Phase am Rotationsverdampfer abgezogen, der Rückstand in 100 ml Aceton aufgenommen und eine Spatelspitze p-Toluolsulfonsäure zugesetzt. Nachdem die Mischung 8 h am Rückfluß gekocht hat, läßt man sie abkühlen, destilliert das Aceton ab, nimmt den Rückstand in Ether auf, versetzt mit 100 ml 1N Natronlauge, trennt die organische Phase ab und extrahiert mit Ether. Nach Vereinigen und Trocknen der organischen Phasen über Natriumsulfat wird eingeengt und das Produkt durch Säulenchromatographie gereinigt. Man erhält 5,4 g der Titelverbindung als gelbes Öl. [DC (Nanoplatten, Petrolether/Essigsäureethylester 6:4) Rf=0,41].

### A2. 4-Hydroxy-4-(2,3-dihydro-7-methoxy-benzofuran-2-spiro-1'-cyclopentan-4-yl)cyclohexanonmonoethylenketal

Zu einer Lösung von 9,5 g (0,034 mol) der nach A4 hergestellten Verbindung in 100 ml THF gibt man bei -78°C tropfenweise unter Stickstoff 21,2 ml (0,036 mol) n-Butyllithium und rührt bei dieser Temperatur noch 0,5 h weiter. Anschließend tropft man eine Lösung von 5,3 g (0,034 mol) Cyclohexandionmonoethylenketal bei -78°C zu und rührt anschließend noch 2 h bei -70°C. Nach Aufwärmen auf RT versetzt man mit 100 ml destilliertem Wasser und neutralisiert mit 1N Salzsäure. Es wird von der organischen Phase abgetrennt und die wäßrige Phase mit Methylenchlorid zusätzlich extrahiert. Die organischen Phasen werden vereinigt und über Natriumsulfat getrocknet, eingeengt und über Säulenchromatographie gereinigt. Man erhält 7,1 g der Titelverbindung als gelbes Öl. [DC (Petrolether/Essigsäureethylester 6:4) Rf=0,16].

### A3. 2,3-Dihydro-7-methoxybenzofuran-2-spiro-1'-cyclopentan-4-boronsäure

Zu einer Lösung von 6,5 g 4-Brom-2,3-dihydro-7-methoxybenzofuran-2-spiro1'-cyclopentan in 100 ml abs. THF tropft man bei -78°C unter Stickstoff 16,6 ml einer 1,6 N Butyllithiumlösung in Hexan. Es wird 2 h bei -78°C gerührt und dann 7,7 ml Borsäuretrimethylester zugetropft und innerhalb von 2 h auf RT erwärmt. Nach Zugabe von 75 ml 1 N Salzsäurelösung wird über Nacht gerührt, dann die Phasen getrennt und die wäßrige Phase noch 2 x mit Diethylether extrahiert. Die vereinigten organischen Phasen werden mit Wasser und gesättigter NaCl-Lösung gewaschen und über Magnesiumsulfat getrocknet. Nach Filtrieren, Einengen und Trocknen im Vakuum erhält man 5,5 g der Titelverbindung als Rohprodukt, das ohne weitere Reinigung weiterverwendet wird.

### A4. 4-Brom-2,3-dihydro-7-methoxybenzofuran-2-spiro-1'-cyclopentan

Zu einer Lösung von 8,4 g (0,03 mol) der nach A5 hergestellten Verbindung in 100 ml absolutem Toluol gibt man 9,0 g Amberlyst 15 und rührt die Mischung für 10 h bei 100°C. Nach Abkühlen der Mischung wird vom H⁺-Ionenaustauscher abfiltriert und mit 100 ml Methanol nachgewaschen. Nach Einengen der organischen Phase und Säulenchromatographie des erhaltenen Rückstandes erhält man 7,4 g der Titelverbindung als gelbes Öl. [DC (Petrolether/Essigsäureethylester 6:4) Rf=0,72].

### A5. 2-(Cyclopent-1-enylmethyl)-3-hydroxy-4-methoxybrombenzol

Zu einer Suspension von 26,5 g (0,074 mol) Methyltriphenylphosphoniumbromid in 200 ml absolutem THF gibt man bei -78°C unter Stickstoff tropfenweise 52,1 ml (0,082 mol) n-Butyllithium (1,6 N in Hexan). Anschließend wird die Suspension auf -30°C erwärmt, wobei die Suspension in Lösung geht. Nach erneutem Abkühlen auf -70°C tropft man eine Lösung von 19,2 g (0,067 mol) der nach A6 hergestellten Verbindung in 200 ml absolutem THF unter Stickstoff zu. Anschließend wird auf -10°C erwärmt und 5 h bei dieser Temperatur gerührt. [DC (Petrolether/Essigsäureethylester 6:4) Rf (Methylenverbindung) =0,81]. Nach Erwärmung auf RT wird die Mischung von Feststoffen abfiltriert, das Filtrat mit 3 x 200 ml halbgesättigter Natriumchlorid-Lösung und 2 x 200 ml destilliertem Wasser ausgeschüttelt. Nach Vereinigung der organischen Phasen, Trocknen über Natriumsulfat und Einengen bis zur Trockne wird der Rückstand in 50 ml Chinolin aufgenommen und 1 h bei 195-205°C gerührt. Nach Abkühlen der Lösung gibt man 400 ml Ethylether zu und schüttelt das Chinolin mit 4 x 200 ml 2N Salzsäure aus. Die organischen Phasen werden vereinigt, über Natriumsulfat getrocknet und eingeengt. Nach Säulenchromatographie des Rückstandes ergibt sich eine Ausbeute von 8,4 g der Titelverbindung als rotbraunes Öl. [DC (Petrolether/Essigsäureethylester 6:4) Rf=0,65].

### A6. 4-Methoxy-3-(2-oxocyclopentyloxy)-brombenzol

Zu einer Lösung von 20 g (0,1 mol) 3-Hydroxy-4-methoxybrombenzol in 300 ml absolutem DMF gibt man 17,7 g (0,15 mol) 2-Chlorcyclopentanon und 41,4 g (0,3 mol) Kaliumcarbonat und rührt 12 h bei RT. Nach Abfiltrieren der Feststoffe wird das Filtrat eingeengt, in 500 ml Ethylether aufgenommen und mit 3 x 200 ml destilliertem Wasser ausgeschüttelt. Säulenchromatographie ergibt 21,1 g der Titelverbindung als braunes Öl [DC (Petrolether/Essigsäureethylester 6:4) Rf=0,47].

### Gewerbliche Anwendbarkeit

Die erfindungsgemäßen Verbindungen besitzen wertvolle pharmakologische Eigenschaften, die sie gewerblich verwertbar machen. Als selektive Zyklisch-Nukleotid Phosphodiesterase (PDE) Inhibitoren (und zwar des Typs IV) eignen sie sich einerseits als Bronchialtherapeutika (zur Behandlung von Atemwegsobstruktionen aufgrund ihrer dilatierenden aber auch aufgrund ihrer atemfrequenz- bzw. atemantriebssteigernden Wirkung) und zur Behebung von erektiler Dysfunktion aufgrund der gefäßdilatierenden Wirkung, andererseits jedoch vor allem zur Behandlung von Erkrankungen, insbesondere entzündlicher Natur, z.B. der Atemwege (Asthma-Prophylaxe), der Haut, des Darms, der Augen und der Gelenke, die vermittelt werden durch Mediatoren, wie Histamin, PAF (Plättchen- aktivierender Faktor), Arachidonsäure-Abkömmlinge wie Leukotriene und Prostaglandine, Zytokine, Interleukine, Chemokine, alpha-, beta- und gamma-Interferon, Tumornekrosisfaktor (TNF) oder Sauerstoff-Radikale und Proteasen. Hierbei zeichnen sich die erfindungsgemäßen Verbindungen durch eine geringe Toxizität, eine gute enterale Resorption (hohe Bioverfügbarkeit), eine große therapeutische Breite und das Fehlen wesentlicher Nebenwirkungen aus.

Aufgrund ihrer PDE-hemmenden Eigenschaften können die erfindungsgemäßen Verbindungen in der Human- und Veterinärmedizin als Therapeutika eingesetzt werden, wobei sie beispielsweise zur Behandlung und Prophylaxe folgender Krankheiten verwendet werden können: Akute und chronische (insbesondere entzündliche und allergeninduzierte) Atemwegserkrankungen verschiedener Genese (Bronchitis, allergische Bronchitis, Asthma bronchiale); Dermatosen (vor allem proliferativer, entzündlicher und allergischer Art) wie beispielsweise Psoriasis (vulgaris), toxisches und allergisches Kontaktekzem, atopisches Ekzem, seborrhoisches Ekzem, Lichen simplex, Sonnenbrand, Pruritus im Genitoanalbereich, Alopecia areata, hypertrophe Narben, diskoider Lupus erythematodes, follikuläre und flächenhafte Pyodermien, endogene und exogene Akne, Akne rosacea sowie andere proliferative, entzündliche und allergische Hauterkrankungen; Erkrankungen, die auf einer überhöhten Freisetzung von TNF und Leukotrienen beruhen, so z.B. Erkrankungen aus dem Formenkreis der Arthritis (Rheumatoide Arthritis, Rheumatoide Spondylitis, Osteoarthritis und andere arthritische Zustände), Erkrankungen des Immunsystems (AIDS, Multiple Sklerose), Erscheinungsformen des Schocks [septischer Schock, Endotoxinschock, gram-negative Sepsis, Toxisches Schock-Syndrom und das ARDS (adult respiratory distress syndrom)] sowie generalisierte Entzündungen im Magen-Darm Bereich (Morbus Crohn und Colitis ulcerosa); Erkrankungen, die auf allergischen und/oder chronischen, immunologischen Fehlreaktionen im Bereich der oberen Atemwege (Rachenraum, Nase) und der angrenzenden Regionen (Nasennebenhöhlen, Augen) beruhen, wie beispielsweise allergische Rhinitis/Sinusitis, chronische Rhinitis/Sinusitis, allergische Conjunctivitis sowie Nasenpolypen; aber auch Erkrankungen des Herzens, die durch PDE-Hemmstoffe behandelt werden können, wie beispielsweise Herzinsuffizienz, oder Erkrankungen, die aufgrund der gewebsrelaxierenden Wirkung der PDE-Hemmstoffe behandelt werden können, wie beispielsweise erektile Dysfunktion oder Koliken der Nieren und der Harnleiter im Zusammenhang mit Nierensteinen; oder auch Erkrankungen des ZNS, wie beispielsweise Depressionen oder arteriosklerotische Demenz.

Ein weiterer Gegenstand der Erfindung ist ein Verfahren zur Behandlung von Säugetieren einschließlich Menschen, die an einer der oben genannten Krankheiten erkrankt sind. Das Verfahren ist dadurch gekennzeichnet, daß man dem erkrankten Säugetier eine therapeutisch wirksame und pharmakologisch verträgliche Menge einer oder mehrerer der erfindungsgemäßen Verbindungen verabreicht.

Weiterer Gegenstand der Erfindung sind die erfindungsgemäßen Verbindungen zur Anwendung bei der Behandlung und/oder Prophylaxe der genannten Krankheiten.

Ebenso betrifft die Erfindung die Verwendung der erfindungsgemäßen Verbindungen zur Herstellung von Arzneimitteln, die zur Behandlung und/oder Prophylaxe der genannten Krankheiten eingesetzt werden.

Weiterhin sind Arzneimittel zur Behandlung und/oder Prophylaxe der genannten Krankheiten, die eine oder mehrere der erfindungsgemäßen Verbindungen enthalten, Gegenstand der Erfindung.

Die Arzneimittel werden nach an sich bekannten, dem Fachmann geläufigen Verfahren hergestellt. Als Arzneimittel werden die erfindungsgemäßen Verbindungen (= Wirkstoffe) entweder als solche, oder vorzugsweise in Kombination mit geeigneten pharmazeutischen Hilfsstoffen z.B. in Form von Tabletten, Dragees, Kapseln, Suppositorien, Pflastern, Emulsionen, Suspensionen, Gelen oder Lösungen eingesetzt, wobei der Wirkstoffgehalt vorteilhafterweise zwischen 0,1 und 95 % beträgt.

Welche Hilfsstoffe für die gewünschten Arzneiformulierungen geeignet sind, ist dem Fachmann aufgrund seines Fachwissens geläufig. Neben Lösemitteln, Gelbildnern, Salbengrundlagen und anderen Wirkstoffträgern können beispielsweise Antioxidantien, Dispergiermittel, Emulgatoren, Konservierungsmittel, Lösungsvermittler oder Permeationspromotoren verwendet werden.

Für die Behandlung von Erkrankungen des Respirationstraktes werden die erfindungsgemäßen Verbindungen bevorzugt auch inhalativ appliziert. Hierzu werden diese entweder direkt als Pulver (vorzugsweise in mikronisierter Form) oder durch Vernebeln von Lösungen oder Suspensionen, die sie enthalten, verabreicht. Bezüglich der Zubereitungen und Darreichungsformen wird beispielsweise auf die Ausführungen im Europäischen Patent 163 965 verwiesen.

Für die Behandlung von Dermatosen erfolgt die Anwendung der erfindungsgemäßen Verbindungen insbesondere in Form solcher Arzneimittel, die für eine topische Applikation geeignet sind. Für die Herstellung der Arzneimittel werden die erfindungsgemäßen Verbindungen (= Wirkstoffe) vorzugsweise mit geeigneten pharmazeutischen Hilfsstoffen vermischt und zu geeigneten Arzneiformulierungen weiterverarbeitet. Als geeignete Arzneiformulierungen seien beispielsweise Puder, Emulsionen, Suspensionen, Sprays, Öle, Salben, Fettsalben, Cremes, Pasten, Gele oder Lösungen genannt.

Die erfindungsgemäßen Arzneimittel werden nach an sich bekannten Verfahren hergestellt. Die Dosierung der Wirkstoffe erfolgt in der für PDE-Hemmstoffe üblichen Größenordnung. So enthalten topische Applikationsformen (wie z.B. Salben) für die Behandlung von Dermatosen die Wirkstoffe in einer Konzentration von beispielsweise 0,1-99 %. Die Dosis für die inhalative Applikation beträgt üblicherweise zwischen 0,01 und 0,5 mg/kg. Die übliche Dosis bei systemischer Therapie liegt zwischen 0,05 und 2 mg/kg und Tag.

### Biologische Untersuchungen

Bei der Untersuchung der PDE IV-Hemmung auf zellulärer Ebene kommt der Aktivierung von Entzündungszellen besondere Bedeutung zu. Als Beispiel sei die FMLP (N-formyl-methionyl-leucyl-phenylalanin)-induzierte Superoxid-Produktion von neutrophilen Granulozyten genannt, die als Luminol-verstärkte Chemolumineszenz gemessen werden kann. [Mc Phail LC, Strum SL, Leone PA und Sozzani S, The neutrophil respiratory burst mechanism. In "Immunology Series" **1992**, 57, 47-76; ed. Coffey RG (Marcel Decker, Inc., New York-Basel-Hong Kong)].

Substanzen, welche die Chemolumineszenz sowie die Zytokinsekretion und die Sekretion entzündungssteigernder Mediatoren an Entzündungszellen, insbesonders neutrophilen und eosinophilen Granulozyten hemmen, sind solche, welche die PDE IV hemmen. Dieses Isoenzym der Phosphodiesterase-Familien ist besonders in Granulozyten vertreten. Dessen Hemmung führt zur Erhöhung der intrazellulären zyklischen AMP-Konzentration und damit zur Hemmung der zellulären Aktivierung. Die PDE IV-Hemmung durch die erfindungsgemäßen Substanzen ist damit ein zentraler Indikator für die Unterdrückung von entzündlichen Prozessen. (**Giembycz MA**, Could isoenzyme-selective phosphodiesterase inhibitors render bronchodilatory therapy redundant in the treatment of bronchial asthma?. Biochem Pharmacol **1992**, 43, 2041-2051; **Torphy TJ** et al., Phosphodiesterase inhibitors: new opportunities for treatment of asthma. Thorax **1991**, 46, 512-523; **Schudt C** et al., Zardaverine: a cyclic AMP PDE III/IV inhibitor. In "New Drugs for Asthma Therapy", 379-402, Birkhäuser Verlag Basel 1991; **Schudt C** et al., Influence of selective phosphodiesterase inhibitors on human neutrophil functions and levels of cAMP and Caᵢ. Naunyn-Schmiedebergs Arch Pharmacol **1991,** 344, 682-690; **Nielson CP** et al., Effects of selective phosphodiesterase inhibitors on polymorphonuclear leukocyte respiratory burst. J Allergy Clin Immunol **1990,** 86, 801-808; **Schade** et al., The specific type III and IV phosphodiesterase inhibitor zardaverine suppress formation of tumor necrosis factor by macrophages. European Journal of Pharmacology **1993,** 230, 9-14).

### 1. Hemmung der PDE IV-Aktivität

### Methodik

Der Aktivitätstest wurde nach der Methode von Bauer und Schwabe durchgeführt, die auf Mikrotiterplatten adaptiert wurde (Naunyn-Schmiedeberg's Arch. Pharmacol. 311, 193-198, 1980). Hierbei erfolgt im ersten Schritt die PDE-Reaktion. In einem zweiten Schritt wird das entstandene 5'-Nukleotid durch eine 5'-Nukleotidase des Schlangengiftes von ophiophagus hannah (King Cobra) zum ungeladenen Nukleosid gespalten. Im dritten Schritt wird das Nukleosid auf Ionenaustauschsäulen vom verbliebenen geladenen Substrat getrennt. Die Säulen werden mit 2 ml 30 mM Ammonium formiat (pH 6,0) direkt in Minivials eluiert, in die noch 2 ml Szintillatorflüssigkeit zur Zählung gegeben wird.

Die für die erfindungsgemäßen Verbindungen ermittelten Hemmwerte ergeben sich aus der folgenden Tabelle A, in der die Nummern der Verbindungen den Nummern der Beispiele entsprechen.

**Tabelle A**

| Hemmung der PDE IV-Aktivität | |
|---|---|
| Verbindung | -log IC₅₀ |
| 1 | 6,99 |
| 2 | 7,84 |
| 3 | 7,98 |
| 4 | 6,90 |
| 5 | 8,08 |

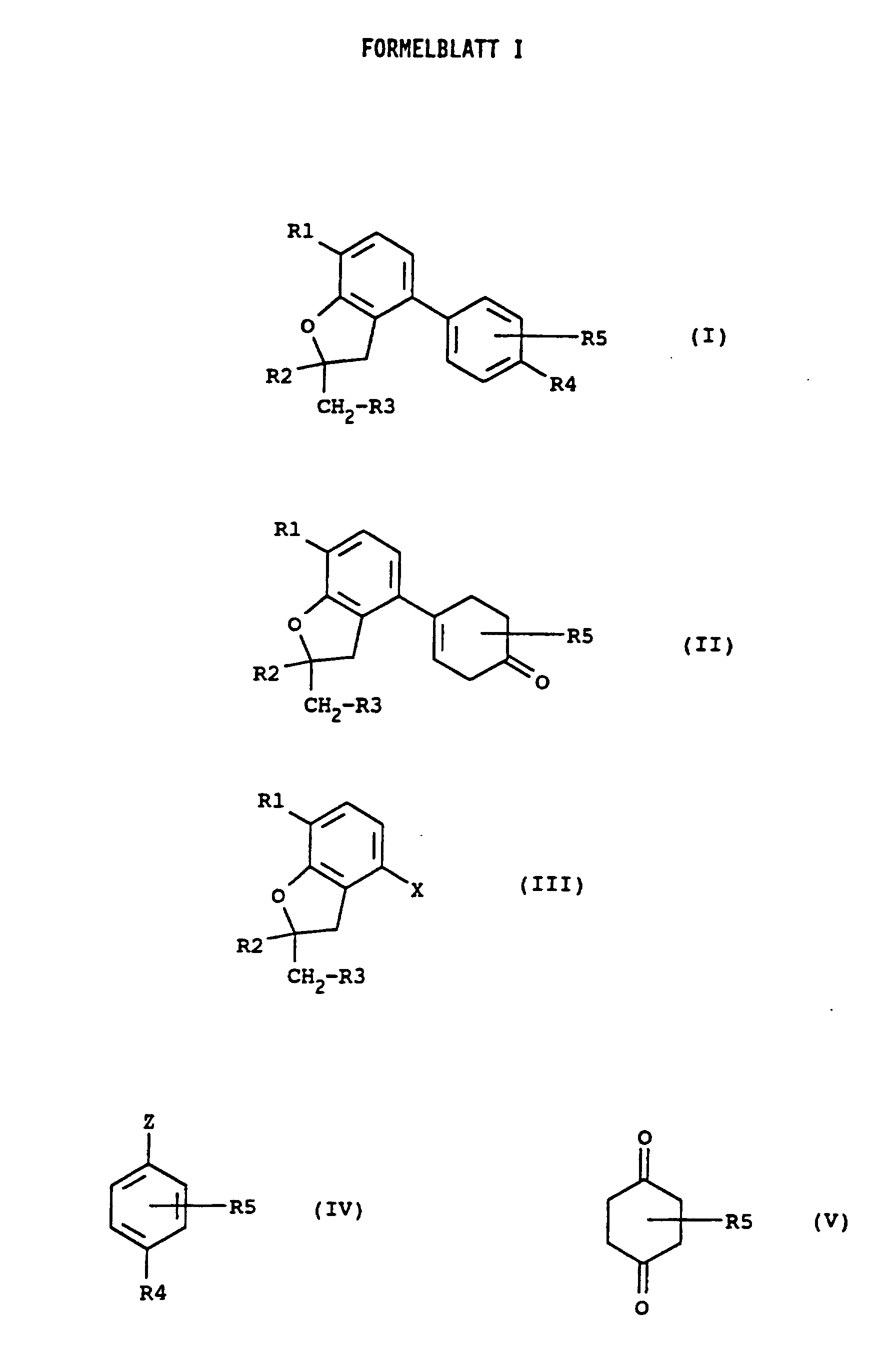

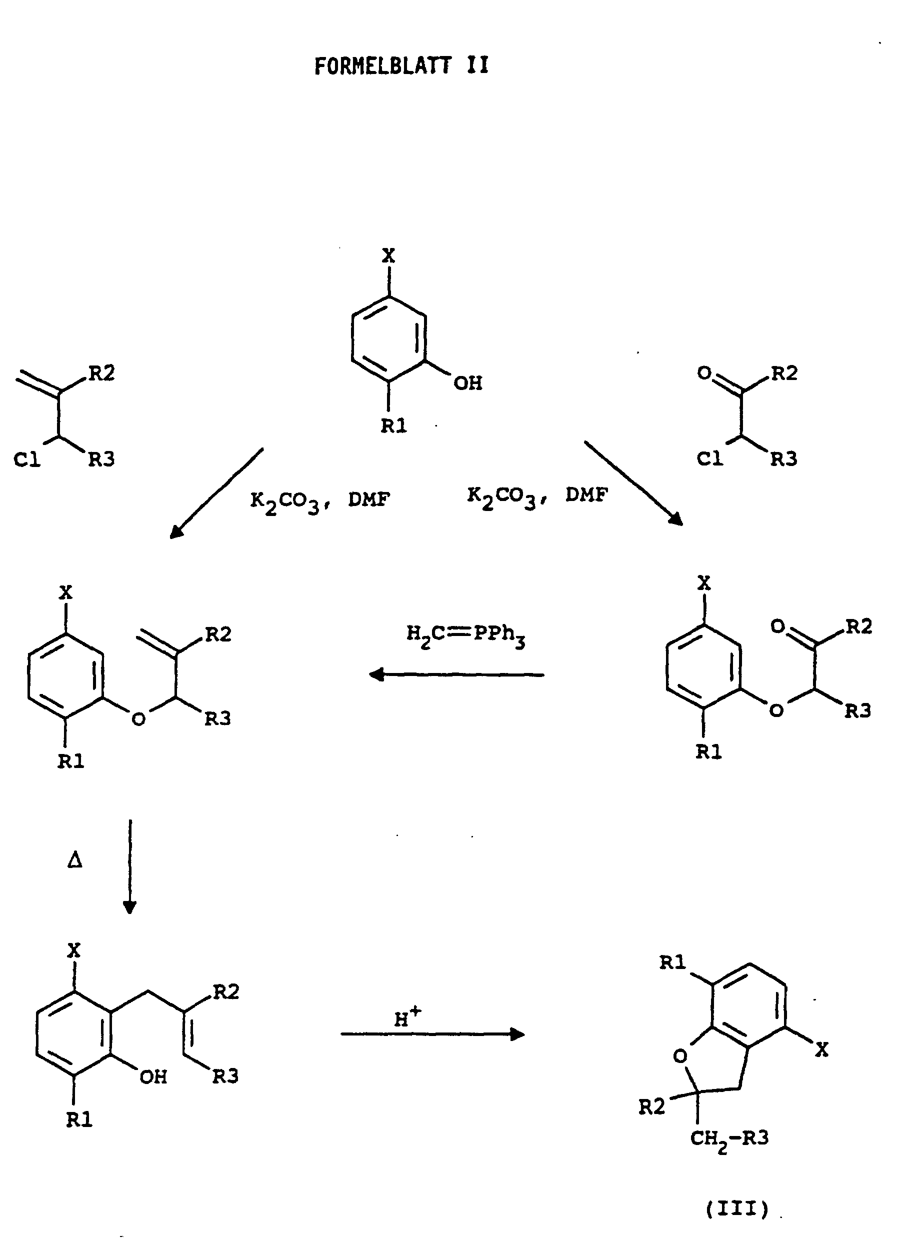

## Patentansprüche

1. Verbindungen der Formel I 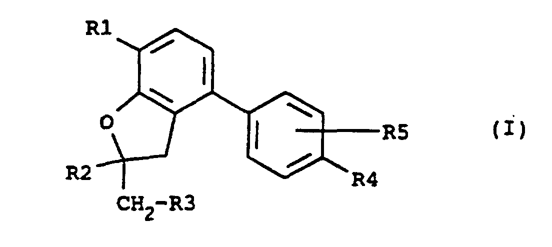 worin
R1 1-6C-Alkoxy, 3-7C-Cycloalkoxy, 3-7C-Cycloalkylmethoxy, Benzyloxy oder ganz oder teilweise durch Fluor substituiertes 1-4C-Alkoxy bedeutet,
R2 1-4C-Alkyl und
R3 Wasserstoff oder 1-4C-Alkyl bedeutet,
oder
R2 und R3 gemeinsam und unter Einschluß der beiden Kohlenstoffatome, an die sie gebunden sind, einen 5-, 6- oder 7-gliedrigen, gewünschtenfalls durch ein Sauerstoffatom unterbrochenen Kohlenwasserstoffring darstellen,
R4 Wasserstoff, Hydroxy, Carboxyl, 1-4C-Alkoxycarbonyl, Amino, Mono- oder Di-1-4C-alkylamino, 1-4C-Alkylcarbonylamino, 1-4C-Alkylsulfonylamino, Trifluormethylsulfonylamino, Cyanamino, Nitro, Cyano, Mono- oder Di-1-4C-alkylaminocarbonyl, 5-Tetrazolyl oder Carbamoyl und
R5 Wasserstoff, Hydroxy, 1-4C-Alkoxy oder Halogen bedeutet und
wobei mindestens einer der Reste R3, R4 und R5 eine von Wasserstoff verschiedene Bedeutung hat,
und deren Salze.

2. Verbindungen der Formel I nach Anspruch 1, in denen
R1 1-4C-Alkoxy, 3-5C-Cycloalkoxy, 3-5C-Cycloalkylmethoxy oder ganz oder teilweise durch Fluor substituiertes 1-4C-Alkoxy bedeutet,
R2 1-4C-Alkyl und
R3 Wasserstoff oder 1-4C-Alkyl bedeutet,
oder
R2 und R3 gemeinsam und unter Einschluß der beiden Kohlenstoffatome, an die sie gebunden sind, einen Cyclopentan-, Cyclohexan-, Tetrahydrofuran- oder Tetrahydropyranring darstellen,
R4 Wasserstoff, Hydroxy, Carboxyl, 1-2C-Alkoxycarbonyl, Nitro, Cyano, Mono- oder Di-1-4C-alkylaminocarbonyl, 5-Tetrazolyl oder Carbamoyl und
R5 Wasserstoff, Hydroxy, Methoxy oder Ethoxy bedeutet und wobei mindestens einer der Reste R3, R4 und R5 eine von Wasserstoff verschiedene Bedeutung hat,
und deren Salze.

3. Verbindungen der Formel I nach Anspruch 1, in denen
R1 1-4C-Alkoxy, 3-5C-Cycloalkoxy oder ganz oder teilweise durch Fluor substituiertes 1-2C-Alkoxy bedeutet,
R2 und R3 gemeinsam und unter Einschluß der beiden Kohlenstoffatome, an die sie gebunden sind, einen Cyclopentan- oder Cyclohexanring darstellen,
R4 Wasserstoff, Hydroxy, Carboxyl, 1-2C-Alkoxycarbonyl, Cyano, Mono- oder Di-1-4C-alkylaminocarbonyl, 5-Tetrazolyl oder Carbamoyl und
R5 Wasserstoff bedeutet,
und deren Salze.

4. Verbindung der Formel I nach Anspruch 1, in denen
R1 Methoxy,
R2 und R3 gemeinsam und unter Einschluß der beiden Kohlenstoffatome, an die sie gebunden sind, einen Cyclopentanring darstellen,
R4 Hydroxy, Carboxyl, Cyano, Mono-1-4C-alkylaminocarbonyl, 5-Tetrazolyl oder Carbamoyl und
R5 Wasserstoff bedeutet,
und deren Salze.

5. Verfahren zur Herstellung der Verbindungen der Formel I und ihrer Salze, **dadurch gekennzeichnet, daß** man
a) Verbindungen der Formel II, 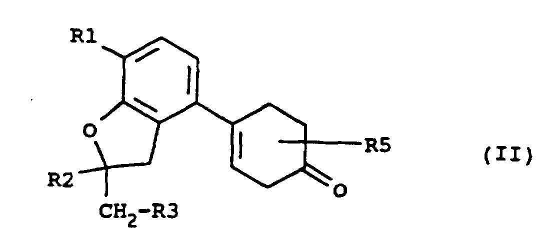 in denen R1, R2, R3 und R5 die in Anspruch 1 angegebenen Bedeutungen haben, katalytisch dehydriert oder daß man
b) Verbindungen der Formel III, 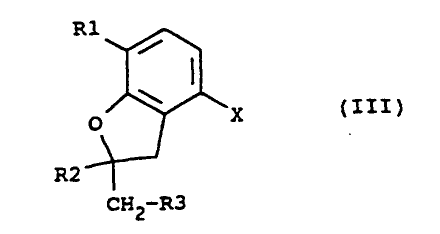 in denen R1, R2 und R3 die in Anspruch 1 genannten Bedeutungen besitzen und X die Gruppe -B(OH)₂ bedeutet mit einer Verbindung der Formel IV, 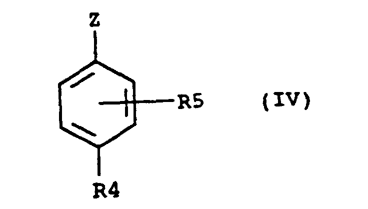 worin R4 und R5 die in Anspruch 1 genannten Bedeutungen besitzen und Z Halogen bedeutet, katalytisch kuppelt oder daß man
c) Verbindungen der Formel I, in denen R1, R2, R3 und R5 die in Anspruch 1 angegebenen Bedeutungen haben und R4 Carboxyl bedeutet, mit Aminen der Formel H-N(R11)R12, worin R11 und R12 unabhängig voneinander Wasserstoff oder 1-4C-Alkyl bedeuten, umsetzt, oder daß man
d) zur Herstellung von Verbindungen der Formel I, in denen R1, R2, R3 und R5 die in Anspruch 1 angegebenen Bedeutungen haben und R4 Cyano bedeutet, entsprechende Verbindungen der Formel I worin R4 Carbamoyl bedeutet dehydratisiert, oder daß man
e) zur Herstellung von Verbindungen der Formel I, in denen R1, R2, R3 und R5 die in Anspruch 1 angegebenen Bedeutungen haben und R4 5-Tetrazolyl bedeutet, entsprechende Verbindungen der Formel I worin R4 Cyano bedeutet mit einem Azidderivat cycloaddiert,
und daß man gewünschtenfalls anschließend nach a), b), c), d) oder e) erhaltene Verbindungen der Formel I in ihre Salze überführt, oder daß man erhaltene Salze der Verbindungen der Formel I in die freien Verbindungen überführt.

6. Arzneimittel enthaltend eine oder mehrere Verbindungen nach Anspruch 1 zusammen mit üblichen pharmazeutischen Hilfs- und/oder Trägerstoffen.

7. Verbindung nach Anspruch 1 zur Anwendung bei der Behandlung von Krankheiten.

8. Verwendung von Verbindungen nach Anspruch 1 zur Herstellung von Arzneimitteln.

9. Verwendung von Verbindungen nach Anspruch 1 zur Herstellung von Arzneimitteln für die Behandlung von Atemwegserkrankungen.

10. Verwendung von Verbindungen nach Anspruch 1 zur Herstellung von Arzneimitteln für die Behandlung von Dermatosen.

## Claims

1. Compounds of the formula I 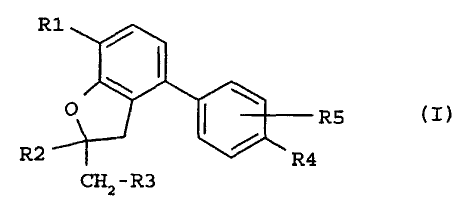 in which
R1 is 1-6C-alkoxy, 3-7C-cycloalkoxy, 3-7C-cycloalkylmethoxy, benzyloxy or 1-4C-alkoxy which is completely or partially substituted by fluorine,
R2 is 1-4C-alkyl and
R3 is hydrogen or 1-4C-alkyl,
or
R2 and R3, together and including the two carbon atoms to which they are bonded, are a 5-, 6- or 7-membered hydrocarbon ring, if desired interrupted by an oxygen atom,
R4 is hydrogen, hydroxyl, carboxyl, 1-4C-alkoxycarbonyl, amino, mono- or di-1-4C-alkylamino, 1-4C-alkylcarbonylamino, 1-4C-alkylsulphonylamino, trifluoromethylsulphonylamino, cyanoamino, nitro, cyano, mono- or di-1-4C-alkylaminocarbonyl, 5-tetrazolyl or carbamoyl and
R5 is hydrogen, hydroxyl, 1-4C-alkoxy or halogen and where at least one of the radicals R3, R4 and R5 has a meaning other than hydrogen,
and their salts.

2. Compounds of the formula I according to Claim 1, in which
R1 is 1-4C-alkoxy, 3-5C-cycloalkoxy, 3-5C-cycloalkylmethoxy or 1-4C-alkoxy which is completely or partially substituted by fluorine,
R2 is 1-4C-alkyl and
R3 is hydrogen or 1-4C-alkyl,
or
R2 and R3, together and including the two carbon atoms to which they are bonded, are a cyclopentane, cyclohexane, tetrahydrofuran or tetrahydropyran ring,
R4 is hydrogen, hydroxyl, carboxyl, 1-2C-alkoxycarbonyl, nitro, cyano, mono- or di-1-4C-alkyl-aminocarbonyl, 5-tetrazolyl or carbamoyl and
R5 is hydrogen, hydroxyl, methoxy or ethoxy and
where at least one of the radicals R3, R4 and R5 has a meaning other than hydrogen,
and their salts.

3. Compounds of the formula I according to Claim 1, in which
R1 is 1-4C-alkoxy, 3-5C-cycloalkoxy or 1-2C-alkoxy which is completely or partially substituted by fluorine,
R2 and R3, together and including the two carbon atoms to which they are bonded, are a cyclopentane or cyclohexane ring,
R4 is hydrogen, hydroxyl, carboxyl, 1-2C-alkoxycarbonyl, cyano, mono- or di-1-4C-alkyl-aminocarbonyl, 5-tetrazolyl or carbamoyl and
R5 is hydrogen,
and their salts.

4. Compounds of the formula I according to Claim 1, in which
R1 is methoxy,
R2 and R3, together and including the two carbon atoms to which they are bonded, are a cyclopentane ring,
R4 is hydroxyl, carboxyl, cyano, mono-1-4C-alkylaminocarbonyl, 5-tetrazolyl or carbamoyl and
R5 is hydrogen,
and their salts.

5. Process for the preparation of the compounds of the formula I and their salts, **characterized in that**
a) compounds of the formula II 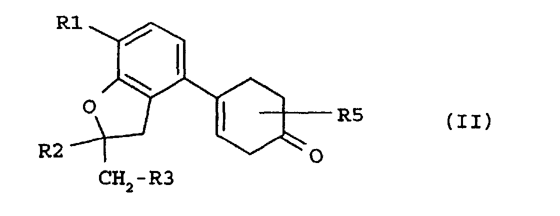 in which R1, R2, R3 and R5 have the meanings indicated in Claim 1, are catalytically dehydrogenated or **in that**
b) compounds of the formula III 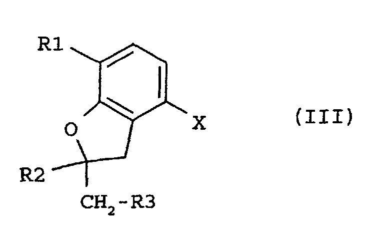 in which R1, R2 and R3 have the meanings mentioned in Claim 1 and X is the group -B(OH)₂ are catalytically coupled with a compound of the formula IV 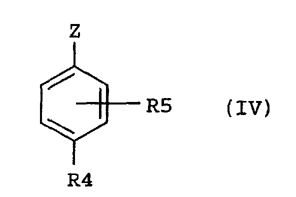 in which R4 and R5 have the meanings mentioned in Claim 1 and Z is halogen, or **in that**
c) compounds of the formula I in which R1, R2, R3 and R5 have the meanings indicated in Claim 1 and R4 is carboxyl, are reacted with amines of the formula H-N(R11)R12, in which R11 and R12 independently of one another are hydrogen or 1-4C-alkyl, or **in that**
d) for the preparation of compounds of the formula I in which R1, R2, R3 and R5 have the meanings indicated in Claim 1 and R4 is cyano, corresponding compounds of the formula I in which R4 is carbamoyl are dehydrated, or **in that**
e) for the preparation of compounds of the formula I in which R1, R2, R3 and R5 have the meanings indicated in Claim 1 and R4 is 5-tetrazolyl, corresponding compounds of the formula I in which R4 is cyano are subjected to cycloaddition with an azide derivative,
and **in that**, if desired, compounds of the formula I obtained according to a), b), c), d) or e) are then converted into their salts, or **in that** salts of the compounds of the formula I obtained are converted into the free compounds.

6. Medicaments comprising one or more compounds according to Claim 1 together with customary pharmaceutical auxiliaries and/or excipients.

7. Compound according to Claim 1 for use in the treatment of illnesses.

8. Use of compounds according to Claim 1 for the production of medicaments.

9. Use of compounds according to Claim 1 for the production of medicaments for the treatment of airway disorders.

10. Use of compounds according to Claim 1 for the production of medicaments for the treatment of dermatoses.

## Revendications

1. Composés de formule I : 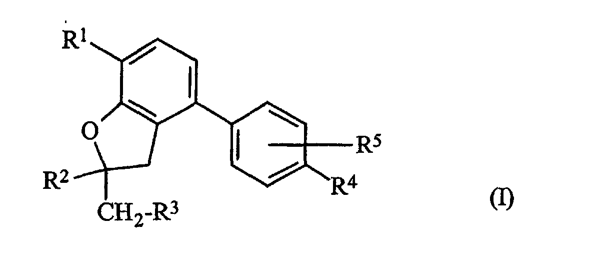 dans laquelle
R1 représente un groupe alcoxy en C₁ à C₆, un groupe cycloalcoxy en C₃ à C₇, un groupe cycloalkyl(C₃ à C₇)méthoxy, un groupe benzyloxy ou un groupe alcoxy en C₁ à C₄ totalement ou partiellement substitué par du fluor,
R2 représente un groupe alkyle en C₁ à C₄ et
R3 représente un atome d'hydrogène ou un groupe alkyle en C₁ à C₄,
ou bien
R2 et R3 forment ensemble et avec les deux atomes de carbone auxquels ils sont liés, un cycle hydrocarboné à 5, 6 ou 7 chaînons, éventuellement interrompu par un atome d'oxygène,
R4 représente un atome d'hydrogène ou un groupe hydroxyle, carboxyle, alcoxy(C₁ à C₄)carbonyle, amino, monoalkyt(C₁ à C₄)amino, di-alkyl(C₁ à C₄)amino, alkyl(C₁ à C₄)carbonylamino, alkyl(C₁ à C₄)sulfonylamino, trifluorométhylsulfonylamino, cyanamino, nitro, cyano, monoalkyl(C₁ à C₄)aminocarbonyle, dialkyl(C₁ à C₄)aminocarbonyle, 5-tétrazolyle ou carbamoyle, et
R5 représente un atome d'hydrogène ou d'halogène ou un groupe hydroxyle ou alcoxy en C₁ à C₄, au moins l'un des groupes R3, R4 et R5 ayant une signification différente de celle de l'atome d'hydrogène,
et les sels de ces composés.

2. Composés de formule I selon la revendication 1, pour lesquels:
R1 représente un groupe alcoxy en C₁ à C₄, un groupe cycloalcoxy en C₃ à C₅, un groupe cycloalkyl(C₃ à C₅)méthoxy ou un groupe alcoxy en C₁ à C₄ totalement ou partiellement substitué par du fluor,
R2 représente un groupe alkyle en C₁ à C₄ et
R3 représente un atome d'hydrogène ou un groupe alkyle en C₁ à C₄,
ou bien
R2 et R3 forment ensemble et avec les deux atomes de carbone auxquels ils sont liés, un cycle cyclopentane, cyclohexane, tétrahydrofurane ou tétrahydropyrane,
R4 représente un atome d'hydrogène ou un groupe hydroxyle, carboxyle, alcoxy(C₁ à C₂)carbonyle, nitro, cyano, monoalkyl(C₁ à C₄)aminocarbonyle, dialkyl(C₁ à C₄)aminocarbonyle, 5-tétrazolyle ou carbamoyle, et
R5 représente un atome d'hydrogène ou un groupe hydroxyle, méthoxy ou éthoxy, au moins l'un des groupes R3, R4 et R5 ayant une signification différente de celle de l'atome d'hydrogène,
et les sels de ces composés.

3. Composés de formule I selon la revendication 1, pour lesquels :
R1 représente un groupe alcoxy en C₁ à C₄, un groupe cycloalcoxy en C₃ à C₅ ou un groupe alcoxy en C₁ à C₂ totalement ou partiellement substitué par du fluor,
R2 et R3 forment ensemble et avec les deux atomes de carbone auxquels ils sont liés, un cycle cyclopentane ou cyclohexane,
R4 représente un atome d'hydrogène ou un groupe hydroxyle, carboxyle, atcoxy(C₁ à C₂)carbonyle, cyano, monoalkyl(C₁ à C₄)aminocarbonyle, di-alkyl(C₁ à C₄)aminocarbonyie, 5-tétrazolyle ou carbamoyle, et
R5 représente un atome d'hydrogène,
et leurs sels.

4. Composés de formule I selon la revendication 1, pour lesquels
R1 représente un groupe méthoxy,
R2 et R3 forment ensemble et avec les deux atomes de carbone auxquels ils sont liés, un cycle cyclopentane,
R4 représente un groupe hydroxyle, carboxyle, cyano, monoalkyl(C₁ à C₄)aminocarbonyle, 5-tétrazolyle ou carbamoyle et
R5 représente un atome d'hydrogène,
et leurs sels.

5. Procédé de préparation des composés de formule I et de leurs sels, **caractérisé en ce que** :
a) on déshydrogène catalytiquement les composés de formule II 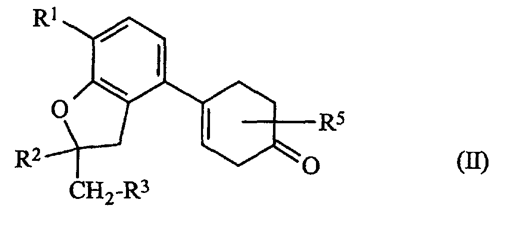 dans laquelle R1, R2, R3 et R5 ont les significations indiquées dans la revendication 1, ou bien
b) on unit catalytiquemsnt les composés de formule III 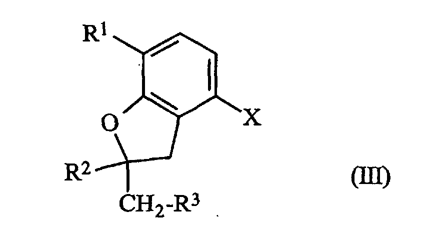 dans laquelle R1, R2 et R3 ont les significations indiquées dans la revendication 1 et X représente le groupe -B(OH)₂, à un composé de formule IV 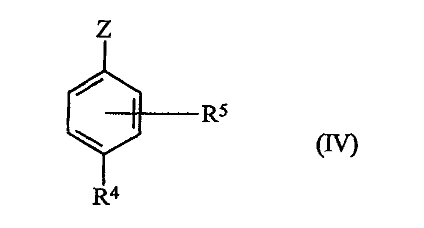 dans laquelle R4 et R5 ont les significations indiquées dans la revendication 1 et Z représente un atome d'halogène, ou bien
c) on fait réagir les composés de formule I pour lesquels R1, R2, R3 et R5 ont les signfications indiquées dans la revendication 1 et R4 représente un groupe carboxyle, avec des amines de formule H-N(R11)R12 dans laquelle R11 et R12 représentent chacun indépendamment un atome d'hydrogène ou un groupe alkyle en C₁ à C₄,
ou bien
d) pour la préparation des composés de formule I pour lesquels R1, R2, R3 et R5 ont les significations indiquées dans la revendication 1 et R4 représente un groupe cyano, on déshydrate les composés correspondants de formule I pour lesquels R4 représente un groupe carbamoyle, ou bien
e) pour la préparation des composés de formule I pour lesquels R1, R2, R3 et R5 ont les significations indiquées dans la revendication 1 et R4 représente le groupe 5-tétrazolyle, on fixe par addition, avec formation de cycle, un dérivé de type azide sur les composés correspondants de formule I pour lesquels R4 représente un groupe cyano,
et **en ce qu'**on transforme ensuite éventuellement les composés de formule I obtenus selon a), b), c), d) ou e) en leurs sels, ou bien on transforme ensuite éventuellement les sels obtenus des composés de formule I en les composés libres.

6. Médicament contenant un ou plusieurs composés selon la revendication 1 ainsi que des produits auxiliaires et/ou supports pharmaceutiques habituels.

7. Composé selon la revendication 1 qui est destiné à être utilisé dans le traitement de maladies.

8. Utilisation des composés selon la revendication 1 pour la préparation de médicaments.

9. Utilisation des composés selon la revendication 1 pour la préparation de médicaments destinés au traitement des affections des voies respiratoires.

10. Utilisation des composés selon la revendication 1 pour la préparation de médicaments destinés au traitement des dermatoses.
